# EUROPEAN PATENT APPLICATION

(11) **EP 3 795 701 A1**
(43) Date of publication of application: **24.03.2021**
(21) Application number: 19803624.6
(22) Date of filing: 26.04.2019
(51) Int. Cl.: C13K 13/00, C07H 3/02

(54) **MANNOSE EXTRACTION METHOD**

(30) Priority: 14.05.2018 JP 2018093089; 09.04.2019 JP 2019074156
(71) Applicant: Futamura Kagaku Kabushiki Kaisha, Nakamura-ku Nagoya-shi Aichi 450-0002 (JP); Tokyo Institute of Technology, Tokyo, 152-0033 (JP)
(72) Inventor: HARA Michikazu, Yokohama-shi, Kanagawa 226-0027 (JP); KITA Yusuke, Yokohama-shi, Kanagawa 226-0027 (JP); HAIGE Ryohei, Kure-shi Hiroshima 737-0134 (JP); YAMADA Hirohumi, Kure-shi Hiroshima 737-0134 (JP)
(74) Representative: TBK
(86) International application number: PCT/JP2019/018003
(87) International publication number: WO 2019/220937

(57) **Abstract**

[Objective] To provide a mannose extraction method with which high-purity mannose can be extremely easily extracted by performing a two-stage hydrolysis treatment on a plant-based raw material. [Solving Means] Mannose is extracted from a plant-based raw material by carrying out a first hydrolysis step S1 in which a plant-based raw material M1 and a first acid catalyst A1 are mixed and heated, a separation step S2 in which a reaction product M2 obtained by the first hydrolysis step is separated and recovered, and a second hydrolysis step S3 in which the reaction product obtained by the separation step and a second acid catalyst A2 are mixed and heated.

## Description

### FIELD

The present invention relates to a mannose extraction method, and in particular, relates to an extraction method wherein high-purity mannose is obtained from a plant-based raw material by carrying out a two-stage hydrolysis treatment with acids.

### BACKGROUND

Mannose, which is a type of monosaccharide, has been attracting attention as a functional sugar in recent years. For example, it is related to the activation of macrophages, suppression of infectious diseases, growth of useful intestinal bacteria, etc. (refer to Patent Literature 1 and 2). Further, it is used as a sweetener additive (refer to Patent Literature 3). Thus, the demand for mannose is expanding rapidly in order to satisfy the use thereof in the fields of pharmaceuticals and foods which take advantage of the active effect of mannose.

Currently, mannose itself is produced from polysaccharides such as glucomannan through enzymatic degradation by microorganisms. Thus, it is not easy to improve production efficiency. In addition, manufacturing costs are also a problem. Thus, a more efficient mannose extract method is desired. Mannose is a type of sugar which constitutes the sugar chains of polysaccharides, and it is known that mannose is primarily present in the form of sugar chains on the surface of plant cell walls.

However, unlike amylose and amylopectin which compose starch, sugar chains of glucomannan, etc., cannot be sufficiently eluted because they are not easily dissolved. Thus, although the presence thereof has been confirmed, the sugar chains have not been effectively utilized as a residual component. In view of this point, methods of extracting mannose oligosaccharides from plant cell walls, i.e., food residues, by acid and heat treatment have been proposed (refer to Patent Literature 4 and 5). In Patent Literature 4, sulfuric acid is added to coffee extract, and in Patent Literature 5, acetic acid or formic acid is added, and mannooligosaccharide (mannan oligosaccharide) is extracted by heating.

However, in Patent Literature 4 and 5, extraction is limited to the oligosaccharide stage, and degradation to monosaccharide mannose and extraction thereof are impossible. Further, since the acid used for reaction is a liquid, removal thereof from the oligosaccharide solution is not easy. Since the acid solution must be thrown away after each treatment, there are many problems relating to production efficiency and cost. However, from a series of circumstances, it has been clarified that the use of an acid is effective for degradation of mannose-containing sugar chains of, for example, glucomannan. Thus, improvement in this new acid treatment has been desired.

The inventors proposed a mannose extraction method using a solid acid catalyst instead of the existing liquid acid catalyst, whereby mannose can be extracted from plant-based food residues by degradation to monosaccharides and catalyst separation can be facilitated (refer to Patent Literature 6).

### [CITATION LIST]

### [PATENT LITERATURE]

[PTL 1] Japanese Unexamined Patent Publication (Kokai) No. 2004-159659
[PTL 2] Japanese Unexamined Patent Publication (Kokai) No. 2010-22267
[PTL 3] Japanese Unexamined Patent Publication (Kokai) No. 2001-352936
[PTL 4] Japanese Examined Patent Publication (Kokoku) No. 5-52200
[PTL 5] Japanese Unexamined Patent Publication (Kokai) No. 2011-132187
[PTL 6] Japanese Unexamined Patent Publication (Kokai) No. 2017-000120

### SUMMARY

### [TECHNICAL PROBLEM]

When mannose is extracted, in addition to mannose, galactose is also extracted, and the separation thereof is not easy. In order to extract mannose at a high purity, it may be time-consuming and costly to further separate the galactose and mannose by chromatography or the like.

The present invention has been proposed in light of the situation described above, and provides a mannose extraction method which enables extremely easy extraction of high-purity mannose by subjecting a plant-based raw material to a two-stage hydrolysis treatment.

### [SOLUTION TO PROBLEM]

Specifically, a first invention provides a mannose extraction method, wherein mannose is extracted from a plant-based raw material by carrying out a first hydrolysis step in which the plant-based raw material and a first acid catalyst are mixed and heated, a separation step in which a reaction product obtained by the first hydrolysis step is separated and recovered, and a second hydrolysis step in which the reaction product obtained by the separation step and a second acid catalyst are mixed and heated.

A second invention provides a mannose extraction method, wherein high-purity mannose is extracted from a plant-based raw material by carrying out a first hydrolysis step in which the plant-based raw material, which includes galactomannan, and a first acid catalyst are mixed and heated to break a bond between a galactose structure and a mannose structure in the galactomannan, and a second hydrolysis step in which a reaction product containing the mannose structure separated by breaking from the galactose structure in the first hydrolysis step and a second acid catalyst are mixed and heated to break a bond between mannose units in the mannose structure contained in the reaction product.

A third invention provides a mannose extraction method wherein, in the first or second invention, the acid catalyst of the first hydrolysis step is a weak acid or dilute strong acid.

A fourth invention provides a mannose extraction method wherein, in the third invention, the acid catalyst of the first hydrolysis step is any of citric acid, acetic acid, oxalic acid, dilute sulfuric acid, and dilute hydrochloric acid.

A fifth invention provides a mannose extraction method wherein, in any of the first to fourth inventions, in the first hydrolysis step, heating is carried out for 3 to 72 hours under temperature conditions of 90 to 160 °C, and the ratio of the amount of mannose to the sum of the amount of mannose and the amount of galactose in an extract after the second hydrolysis step has completed is 80% or more.

A sixth invention provides a mannose extraction method wherein, in the fifth invention, the ratio of the amount of galactose to the sum of the amount of galactose and the amount of mannose in a solution obtained in the first hydrolysis step is 38% or more.

A seventh invention provides a mannose extraction method wherein, in any of the first to sixth inventions, the acid catalyst of the second hydrolysis step is any of a weak acid, a dilute strong acid, a strong acid, or a solid acid.

An eighth invention provides a mannose extraction method wherein, in the seventh invention, the acid catalyst of the second hydrolysis step is any of citric acid, acetic acid, oxalic acid, dilute sulfuric acid, dilute hydrochloric acid, sulfuric acid, hydrochloric acid, a wood solid acid catalyst obtained by introducing a sulfo group into a carbonized material derived from a wood-based raw material and carrying out sulfonation, or a resin solid acid catalyst obtained by introducing a sulfo group into a phenol resin and carrying out sulfonation.

A ninth invention provides a mannose extraction method wherein, in any of the first to eighth inventions, in the second hydrolysis step, heating is carried out for 1 to 24 hours under temperature conditions of 90 to 160 °C.

A tenth invention provides a mannose extraction method wherein, in any of the first to ninth inventions, the plant-based raw material is coffee bean extraction residue.

An eleventh invention provides a mannose extraction method wherein, in any of the first to ninth inventions, the plant-based raw material is konjac potato.

### [ADVANTAGEOUS EFFECTS OF INVENTION]

According to the mannose extraction method of the first invention, since mannose is extracted from a plant-based raw material by carrying out a first hydrolysis step in which the plant-based raw material and a first acid catalyst are mixed and heated, a separation step in which a reaction product obtained by the first hydrolysis step is separated and recovered, and a second hydrolysis step in which the reaction product obtained by the separation step and a second acid catalyst are mixed and heated, high-purity mannose can be extremely easily extracted from the plant-based raw material and production costs can be reduced.

According to the mannose extraction method of the second invention, since high-purity mannose is extracted from a plant-based raw material by carrying out a first hydrolysis step in which the plant-based raw material, which includes galactomannan, and a first acid catalyst are mixed and heated to break a bond between a galactose structure and a mannose structure in the galactomannan, and a second hydrolysis step in which a reaction product containing the mannose structure separated by breaking from the galactose structure in the first hydrolysis step and a second acid catalyst are mixed and heated to break a bond between mannose units in the mannose structure contained in the reaction product, high-purity mannose can be extremely easily extracted from the plant-based raw material and production costs can be reduced.

According to the mannose extraction method of the third invention, since the acid catalyst of the first hydrolysis step is a weak acid or dilute strong acid in the first or second invention, adjustment of the hydrolysis reaction of the plant-based raw material is easy and the first hydrolysis step can be easily and reliably carried out.

According to the mannose extraction method of the fourth invention, since the acid catalyst of the first hydrolysis step is any of citric acid, acetic acid, oxalic acid, dilute sulfuric acid, and dilute hydrochloric acid in the third invention, high-purity mannose can be extremely easily extracted.

According to the mannose extraction method of the fifth invention, since, in the first hydrolysis step, heating is carried out for 3 to 72 hours under temperature conditions of 90 to 160 °C, and the ratio of the amount of mannose to the sum of the amount of mannose and the amount of galactose in an extract after the second hydrolysis step has completed is 80% or more in any of the first to fourth inventions, efficient reaction promotion and stability of the purity of the obtained mannose are achieved.

According to the mannose extraction method of the sixth invention, since the ratio of the amount of galactose to the sum of the amount of galactose and the amount of mannose in a solution obtained in the first hydrolysis step is 38% or more in the fifth invention, mannose can be extracted at a high purity.

According to the mannose extraction method of the seventh invention, since the acid catalyst of the second hydrolysis step is any of a weak acid, a dilute strong acid, a strong acid, or a solid acid in any of the first to sixth inventions, adjustment of the hydrolysis reaction of the reaction product is easy and the second hydrolysis step can be easily and reliably performed.

According to the mannose extraction method of the eighth invention, since the acid catalyst of the second hydrolysis step is any of citric acid, acetic acid, oxalic acid, dilute sulfuric acid, dilute hydrochloric acid, sulfuric acid, hydrochloric acid, a wood solid acid catalyst obtained by introducing a sulfo group into a carbonized material derived from a wood-based raw material and carrying out sulfonation, or a resin solid acid catalyst obtained by introducing a sulfo group into a phenol resin and carrying out sulfonation in the seventh invention, high-purity mannose can be extracted smoothly from the plant-based raw material through catalytic reaction.

According to the mannose extraction method of the ninth invention, since, in the second hydrolysis step, heating is carried out for 1 to 24 hours under temperature conditions of 90 to 160 °C in any of the first to eighth inventions, both efficient reaction promotion and stability of the purity of the obtained mannose can be achieved.

According to the mannose extraction method of the tenth invention, since the plant-based raw material is coffee bean extract reside in any of the first to ninth inventions, food residues can be effectively used, raw material procurement is easy, and homogeneity is high.

According to the mannose extraction method of the eleventh invention, since the plant-based raw material is konjac potato in any of the first to ninth inventions, raw material procurement is easy and homogeneity is high.

### BRIEF DESCRIPTION OF DRAWINGS

FIG. 1 is a schematic process diagram of the mannose extraction method of the present invention.
FIG. 2 is a schematic view of the structure of sugar contained in a plant-based raw material.

### DESCRIPTION OF EMBODIMENTS

The mannose extraction method prescribed in the present invention is a method for obtaining high-purity mannose by subjecting a plant-based raw material to a two-stage hydrolysis treatment. By performing a first hydrolysis step in which the plant-based raw material and a first acid catalyst are mixed and heated and a separation step in which a solution containing the reaction product obtained by the first hydrolysis step and components eluted by the first hydrolysis step are separated, monosaccharides (for example, galactose) other than mannose contained in the plant-based raw material are removed from the plant-based raw material prior to the second hydrolysis step. High-purity mannose is obtained by adding a second acid catalyst to the reaction product obtained by these steps and heating in a second hydrolysis step, and extracting the mannose contained in the reaction product resulting from the second hydrolysis reaction. Thus, since it is not necessary to separate other sugars and mannose by chromatography, high-purity mannose can be extremely easily and economically obtained.

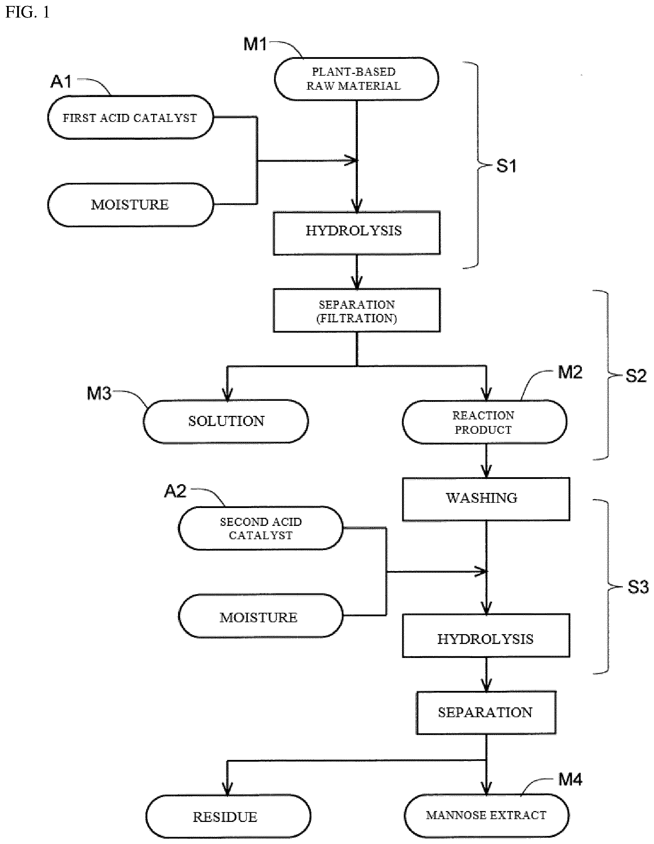

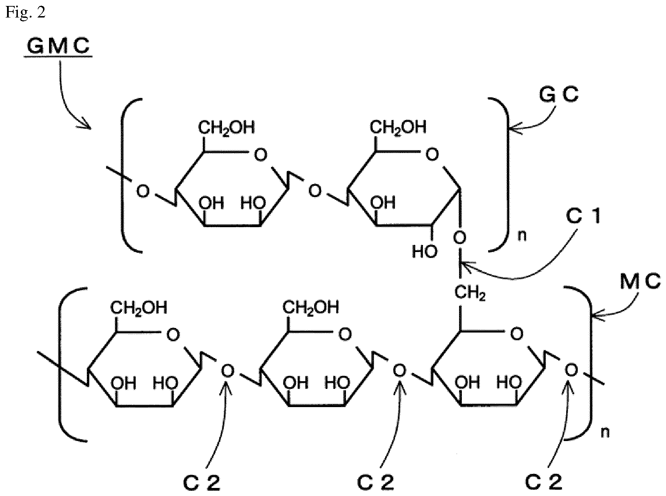

A summary of the mannose extraction method will be described using the schematic process diagram of FIG. 1 and the schematic view of the structure of sugar contained in the raw material of FIG. 2. The plant-based raw material M1 from which mannose is extracted is soy pulp, sake residue, tea extraction residue, coffee bean extraction residue, other residual components which are generated during food processing, or konjac potato. Coffee bean extraction residue is a residue which is generated when water or boiling water is added to roasted coffee beans to extract coffee. Most thereof is treated as food residue products, since coffee drinks are produced in large quantities. Thus, the food residues can be effectively used, raw materials can be easily procured, and the homogeneity of the residue itself is high. Konjac potato is useful because the procurement of raw materials is easy and konjac potato has high homogeneity. Although not included in foods, plant materials such as rice straw, waste timber, waste bamboo, and copra meal are also added to the residue.

The reason why the use of plant-based food residue products is preferable is that the use has the following advantages over mere treatment of residue products. Various sugar chains other than cellulose are present on the cell wall surfaces of plant cells. It is believed that these sugar chains act on cell adhesion between plant cells and maintenance of the shape of the plant body. However, the human body often cannot digest these sugar chain components for nutrition. Thus, though the presence of such sugar chains as unused components is clear, they have not been effectively utilized. For example, mannose is obtained by degradation of sugar chains of, for example, glucomannan to monosaccharides. Thus, coffee bean extraction residue, which is simple and has a high mannose content, is selected as the source of mannose.

The plant-based raw material is crushed (pulverized) to an appropriate size in advance if necessary. As shown in FIG. 2, the sugar structure contained in the coffee bean extraction residue is considered to have a galactomannan structure GMC. The galactose structure GC and the mannose structure MC are linked by an α-1,6-glycoside bond C1. It is considered that the galactose structures GC linked to the mannose structures MC by the α-1,6-glycoside bonds C1 are linked at the ends thereof to galactose and mannose at a ratio of about 1:1. In the mannose structure MC, a plurality of mannose units are considered to be linked by β-1,4-glycoside bonds C2.

As shown in FIG. 1, the mannose extraction method of the present invention includes the first hydrolysis step S1, the separation step S2, and the second hydrolysis step S3. Utilizing the difference in hydrolysis rate between the α-1,6-glycoside bond C1 and the β-1,4-glycoside bond C2, mannose is extracted at a high purity from the plant-based raw material M1. Specifically, the first hydrolysis step S1 is a step in which the α-1,6-glycoside bond C1 having a high hydrolysis rate and linking the galactose structure GC and the mannose structure MC together is broken by subjecting the plant-based raw material M1 to a hydrolysis reaction. When the α-1,6-glycoside bond C1 is broken, the galactose structure GC is eluted into the solution M3, and the mannose structure MC remains in the reaction product M2.

The separation step S2 is a step in which the reaction product M2 and the solution M3 are separated by filtration. The reaction product M2 is then appropriately washed. Further, in the second hydrolysis step S3, the reaction product M2 containing the mannose structure Mc obtained by the first hydrolysis step is subjected to a hydrolysis reaction. As a result, the β-1,4-glycoside bond C2 having a low hydrolysis rate and linking mannose units together in the mannose structure MC is broken, whereby high-purity mannose is obtained.

In the first hydrolysis step S1, an acid catalyst A1 is added to the plant-based raw material, and then mixed and heated. During heating, moisture is appropriately adjusted. In order to promote the reaction of the plant-based raw material M1, it is desirable that moisture be present. However, when there is excessive moisture, the extracted components generated by degradation of the plant-based raw material M1 are diluted. The amount of moisture is suitably adjusted in consideration of this point. In the first hydrolysis step S1, it is sufficient if the plant-based raw material is able to undergo the hydrolysis reaction. Thus, the acid catalyst to be used is not particular limited, and it is believed that a weak acid or a dilute strong acid is preferable. The first hydrolysis step S1 is intended to break the α-1,6-glycoside bond C1 having a high hydrolysis rate. Thus, when a strong acid is used as the acid catalyst A1, adjustment of the reaction temperature and reaction time necessary to perform a proper hydrolysis reaction is not easy. When a weak acid or a dilute strong acid, which has a relatively low hydrolysis performance, is selected, since the reaction temperature and reaction time can be easily adjusted, employee labor is reduced and production efficiency and stability are improved.

The acid catalyst A1 added in the first hydrolysis step S1 may be a weak acid such as citric acid, acetic acid, or oxalic acid, or a dilute strong acid obtained by diluting a strong acid such as sulfuric acid or hydrochloric acid. Since each of these acids has a different hydrolysis performance, even if hydrolysis is performed under the same concentration, temperature, and time conditions, the intent of the first hydrolysis step S 1 cannot be equivalently achieved.

As described above, it is considered that the galactose structures GC are linked (at the ends of the α-1,6-glycoside bonds C1) to galactose and mannose at a ratio of about 1:1. Thus, if the component ratio of mannose and galactose, which are the components eluted in the first hydrolysis step S1, is approximately 1:1, the intent of the first hydrolysis step S1 can be sufficiently achieved. Considering that part of mannose contained in the mannose structure MC may be eluted, it is considered preferable that the type and addition amount of the acid and the heating temperature and heating time be adjusted so that the ratio of the amount of galactose to the sum of the amount of galactose and the amount of mannose contained in the solution M3 is 38% or more.

Among the components contained in the solution M3, when the content ratio of mannose is excessively high, since the mannose contained in the mannose structure is considered to have been eluted, the amount of mannose extracted after the second hydrolysis step S3 is considered to decrease. However, this does not reduce the purity of the extracted mannose. Thus, in the first hydrolysis step S1, the conditions for treating the raw material with the above-mentioned acid catalyst are selected from conditions sufficient to break the α-1,6-glycoside bond C1 of the galactomannan structure GMC contained in the raw material. The range of 38% or more of the ratio of the amount of galactose to the sum of the amount of galactose and the amount of mannose contained in the solution M3 as described above is particularly useful from the viewpoint that the amount of mannose extracted after the second hydrolysis step S3 can be sufficiently ensured.

In the first hydrolysis step S 1, if the reaction temperature at the time of heating is excessively high, the bonds of the mannose structure MC may be broken, or the mannose itself may be deteriorated due to oxidation or degradation. The same is true if the reaction time of the heating is excessively long. Thus, the amount of mannose extracted after the second hydrolysis step S3 may decrease. Conversely, if the reaction temperature is excessively low or the reaction time is excessively short, galactose remains in the reaction product obtained after the first hydrolysis step S1, and it becomes difficult to obtain mannose of a high purity. From the viewpoint of achieving both efficient reaction promotion and stability of the reaction product, a reaction for 3 to 72 hours at a heat temperature range of 90 to 160 °C is considered appropriate. The conditions of reaction temperature and reaction time are preferably adjusted so that the ratio of amount of mannose to the sum of amount of mannose and amount of galactose in the extract after the second hydrolysis step is 80% or more, and the conditions are appropriately determined in accordance with the type of acid catalyst used and the addition amount thereof.

In the first hydrolysis step S1, if the hydrolysis performance of the acid catalyst is high in accordance with the addition amount and type of the acid catalyst, it is considered that the reaction temperature should be lowered and heating should be performed for a short time. Conversely, when the hydrolysis performance of the acid catalyst is low, it is considered preferable to increase the reaction temperature or increase the heating time. The addition amount and type of the acid catalyst and the balance between reaction temperature and reaction time are adjusted so that the ratio of the amount of mannose to the sum of the amount of mannose and the amount of galactose of the extract after the second hydrolysis step has completed is 80% or more. Furthermore, considering the ultimate extraction amount of mannose extracted after the second hydrolysis step S3, the type and addition amount of the acid catalyst and the conditions of the reaction temperature and reaction time are preferably determined so as to achieve a ratio of the amount of galactose to the sum of the amount of galactose and the amount of mannose contained in the solution M3 in the range of 38% or more. If the acid catalyst is any of citric acid, acetic acid, oxalic acid, dilute sulfuric acid, or dilute hydrochloric acid, heating under conditions of, for example, a temperature of 90 to 160 °C and a time of 3 to 72 hours is considered preferable. Since hydrolysis does not readily occur at temperatures of 80 °C or lower, the lower limit of reaction temperature is approximately 90 °C. Further, from the viewpoint of production efficiency, setting the reaction temperature to 120 °C to 140 °C reduces the reaction time, which is more economical.

In the first hydrolysis step S1, the α-1,6-glycoside bond C1 is broken, whereby the mannose and galactose contained in the galactose structure GC are eluted into the solution M3. However, the mannose structure MC remains in the reaction product M2. In the separation step S2, the reaction product M2 generated through the hydrolysis reaction in the first hydrolysis step S1 is separated and recovered from the solution M3 to which the acid catalyst used for reaction and galactose have been eluted. Separation methods such as filtration or centrifugal separation are suitable. As a result of the hydrolysis reaction with the acid catalyst A1 under moist conditions, the galactose and mannose of the galactose structure GC contained in the plant-based raw material M1 are eluted, separated, and removed. If necessary, the reaction product M2 is washed.

In the second hydrolysis step S3, the reaction product M2 and the acid catalyst A2 such as a weak acid, a dilute strong acid, a strong acid, or a solid acid are mixed and heated. The temperature and time conditions are appropriately determined in accordance with the type of the acid catalyst used and the addition amount thereof. Like the reaction temperature and reaction time in the first hydrolysis step S1, the reaction temperature and reaction time are preferably adjusted so that the ratio of the amount of mannose to the sum of the amount of mannose and the amount of galactose in the extract after the second hydrolysis step has completed is 80% or more. The separated reaction product M2 resulting from the separation step S2 contains the mannose structure MC. Thus, by hydrolyzing the β-1,4-glycoside bond C2 linking the mannose units together contained in the solid mannose structure MC, a sugar solution (mannose extract M4) containing high-purity mannose can be obtained. As in the first hydrolysis step S1, the moisture during heating is appropriately adjusted. In the second hydrolysis step S3, after the hydrolysis reaction has completed, mannose extract M4 containing high-purity mannose and reaction residue are separated.

The second hydrolysis step S3 is intended to break the β-1,4-glycoside bond C2, which has a lower hydrolysis rate than the α-1,6-glycoside bond C1. Thus, the hydrolysis reaction is promoted by mixing the acid catalyst A2, which is a stronger acid than the acid catalyst A1 added in the first hydrolysis step A1, into the reaction product M2 or heating the acid catalyst A2, which has the same acidity, at a higher temperature or for a longer time for the reaction.

Among the types of acid catalyst A2 which can be added in the second hydrolysis step S3, examples of strong acid catalysts include sulfuric acid and hydrochloric acid. Examples of acid catalysts of weak acids and dilute strong acids include citric acid, acetic acid, oxalic acid, dilute sulfuric acid, and dilute hydrochloric acid. Further, examples of solid acid catalysts include wood solid acid catalysts obtained by introducing a sulfo group into a carbonized material derived from a wood raw material and carrying out sulfonation, and resin solid acid catalysts obtained by introducing a sulfo group into a phenol resin and carrying out sulfonation. Since each acid has a different hydrolysis performance, it is not possible to achieve the intent of the second hydrolysis step S3 at the same concentration, temperature, and time conditions for all acid catalysts. If the temperature is excessively high or the heating time is excessively long, the mannose itself, which is the target product, may be deteriorated due to oxidation or degradation. Thus, in order to break the β-1,4-glycoside bond C2, it is considered preferable to carry out heating for approximately 1 to 24 hours at a temperature of 90 to 160 °C. Considering the efficient reaction rates of these acids, reaction under high temperature conditions is considered preferable, and heating at 120 to 160 °C for 1 to 6 hours is more suitable.

In particular, the solid acid catalyst is mixed with the plant-based raw material and heated in the presence of moisture, and thereafter the mannose produced by degradation is eluted into the existing moisture. If only water is separated by filtration or centrifugal separation, water containing only mannose can be separated very easily. Thus, the load required for separation after the catalyst reaction is greatly reduced, whereby manufacturing costs can be reduced.

Solid wood acid catalysts are obtained by carbonization of a wood-based raw material to form a carbonized material under temperature conditions such that the wood-based raw material does not burn, and sulfonation to introduce a sulfo group (also referred to as a sulfonic acid group). As the wood-based solid acid catalyst, for example, the solid acid disclosed in Japanese Patent No. 5528036 can be used. Resin solid acid catalysts are obtained by introducing a sulfo group into a raw material phenol resin and carrying out sulfonation. If a resin solid acid catalyst having a high heat resistance is used, the heating temperature in the second hydrolysis step S3 can be increased and the heating time can be reduced. In addition to powdered solid acids, the solid acid can be shaped (processed) into a predetermined shape. Shaping makes the particles larger than in powder form, which facilitates separate and recovery from the reaction liquid.

### EXAMPLES

### [Sample]

The inventors attempted to extract mannose from plant-based raw material using the acid catalysts in the Tables. Deionized water was added to commercially available powdery (milled) coffee beans to produce a slurry having a concentration thereof of 5 wt%, and this slurry was boiled for 30 minutes. After boiling, filtration was repeated 3 times or more to separate the coffee bean extraction residue. The coffee bean extraction residue was dried overnight in a dryer maintained at 105 ± 5 °C and crushed to 0.3 mm or less by a crusher. The coffee bean extraction residue serving as the sample of the plant-based raw material was obtained in this manner. This coffee bean extraction residue served as the sample in each of the experimental examples.

### <Experimental Example 1>

0.05 g of 1.0 wt% citric acid as the first acid catalyst and 5.0 g of deionized water were added to 0.5 g (dry weight) of coffee bean extraction residue in a 15 mL pressure-resistant reaction vessel and reacted for 20 hours while the temperature was maintained at 120 °C (first hydrolysis step S1). After the reaction was complete, the reaction product was separated and recovered using a membrane filter (pore diameter: 0.2 µm) and washed with an excess amount of running deionized water (separation step S2). 0.3 g of 10% (v/v) dilute sulfuric acid as the second acid catalyst and 4.2 g of deionized water were added to 0.3 g of the recovered reaction product and reacted for 1 hour while the temperature was maintained at 140 °C (second hydrolysis step S3). After the reaction was complete, the mixture was chilled, and 9.3 g of deionized water was added to the reaction vessel to dilute the reaction product. The reaction liquid was filtered using a syringe filter (pore diameter: 0.2 µm) to obtain the extract of Experimental Example 1.

### <Experimental Example 2>

A reaction product obtained by the same first hydrolysis step as Experimental Example 1 was separated and recovered, and 0.3 g of 10% (v/v) dilute sulfuric acid as the second acid catalyst and 4.2 g of deionized water were added to 0.3 g of the reaction product and reacted for 3 hours while the temperature was maintained at 120 °C. After the reaction was complete, the reaction product was chilled and 9.3 g of deionized water was added to the reaction vessel to dilute the reaction product. The reaction liquid was filtered using a syringe filter (pore diameter: 0.2 µm) to obtain the extract of Experimental Example 2.

### <Experimental Example 3>

A reaction product obtained by the same first hydrolysis step as Experimental Example 1 was separated and recovered, and 0.3 g of a wood solid acid catalyst (ZP150DH manufactured by Futamura Chemical Co., Ltd.) as the second acid catalyst and 4.2 g of deionized water were added to 0.3 g of solids and reacted for 1 hour while the temperature was maintained at 140 °C. After the reaction was complete, the reaction product was chilled and 9.3 g of deionized water was added to the reaction vessel to dilute the reaction product. The reaction liquid was filtered using a syringe filter (pore diameter: 0.2 µm) to obtain the extract of Experimental Example 3.

### <Experimental Example 4>

The extract of Experimental Example 4 was obtained by the same method as Experimental Example 3 except that the reaction time of the second hydrolysis step was 3 hours.

### <Experimental Example 5>

The extract of Experimental Example 5 was obtained by the same method as Experimental Example 4 except that the reaction temperature of the second hydrolysis step was 120 °C.

### <Experimental Example 6>

The extract of Experimental Example 6 was obtained by the same method as Experimental Example 5 except that the reaction time of the second hydrolysis step was 6 hours.

### <Experimental Example 7>

The extract of Experimental Example 7 was obtained by the same method as Experimental Example 4 except that 0.05 g of 10.0 wt% citric acid as the first acid catalyst of the first hydrolysis step and 5.0 g of deionized water were added and reacted for 24 hours while the temperature was maintained at 90 °C.

### <Experimental Example 8>

The extract of Experimental Example 8 was obtained by the same method as Experimental Example 7 except that the reaction time of the first hydrolysis step was 48 hours.

### <Experimental Example 9>

The extract of Experimental Example 9 was obtained by the same method as Experimental Example 7 except that the reaction time of the first hydrolysis step was 72 hours.

### <Experimental Example 10>

The extract of Experimental Example 10 was obtained by the same method as Experimental Example 7 except that 0.10 g of 20.0 wt% citric acid as the first acid catalyst of the first hydrolysis step and 5.0 g of deionized water were added.

### <Experimental Example 11>

The extract of Experimental Example 11 was obtained by the same method as Experimental Example 7 except that 0.15 g of 30.0 wt% citric acid as the first acid catalyst of the first hydrolysis step and 5.0 g of deionized water were added.

### <Experimental Example 12>

The extract of Experimental Example 12 was obtained by the same method as Experimental Example 4 except that the reaction temperature and reaction time of the first hydrolysis step were 140 °C and 3 hours.

### <Experimental Example 13>

The extract of Experimental Example 13 was obtained by the same method as Experimental Example 4 except that the reaction temperature and reaction time of the first hydrolysis step were 160 °C and 3 hours.

### <Experimental Example 14>

The extract of Experimental Example 14 was obtained by the same method as Experimental Example 4 except that 0.0185 g of 3.7 wt% sulfuric acid as the first acid catalyst of the first hydrolysis step and 5.0 g of deionized water were added.

### <Experimental Example 15>

The extract of Experimental Example 15 was obtained by the same method as Experimental Example 4 except that 0.009 g of 1.8 wt% sulfuric acid as the first acid catalyst of the first hydrolysis step and 5.0 g of deionized water were added.

### <Experimental Example 16>

The extract of Experimental Example 16 was obtained by the same method as Experimental Example 4 except that 0.05 g of 10.0 wt% sulfuric acid as the first acid catalyst of the first hydrolysis step and 5.0 g of deionized water were added.

<Experimental Example 17>

The extract of Experimental Example 17 was obtained by the same method as Experimental Example 4 except that 0.012 g of 2.4 wt% hydrochloric acid as the first acid catalyst of the first hydrolysis step and 5.0 g of deionized water were added.

### <Experimental Example 18>

The extract of Experimental Example 18 was obtained by the same method as Experimental Example 17 except that 0.006 g of 1.2 wt% hydrochloric acid as the first acid catalyst of the first hydrolysis step and 5.0 g of deionized water were added.

### <Experimental Example 19>

The extract of Experimental Example 19 was obtained by the same method as Experimental Example 4 except that 0.005 g of 1.0 wt% acetic acid as the first acid catalyst of the first hydrolysis step and 5.0 g of deionized water were added, and the reaction temperature was 140 °C.

### <Experimental Example 20>

The extract of Experimental Example 20 was obtained by the same method as Experimental Example 19 except that 0.05 g of 10.0 wt% acetic acid as the first acid catalyst of the first hydrolysis step and 5.0 g of deionized water were added.

### <Experimental Example 21>

The extract of Experimental Example 21 was obtained by the same method as Experimental Example 4 except that 0.005 g of 1.0 wt% oxalic acid as the first acid catalyst of the first hydrolysis step and 5.0 g of deionized water were added.

### <Experimental Example 22>

The extract of Experimental Example 22 was obtained by the same method as Experimental Example 21 except that 0.05 g of 10.0 wt% oxalic acid was the first acid catalyst of the first hydrolysis step and 5.0 g of deionized water were added.

As Comparative Examples, mannose extraction treatment was carried out without performing the first hydrolysis step S1. Since the first hydrolysis step S1 was omitted, separation step S2 was also omitted.

### <Comparative Example 1>

0.3 g of a wood solid acid catalyst (ZP150DH manufactured by Futamura Chemical Co., Ltd.) as the (second) acid catalyst and 4.2 g of deionized water were added to 0.3 g (dry weight) of coffee bean extract residue in a 15 mL pressure-resistant reaction vessel and reacted for 3 hours while the temperature was maintained at 140 °C. After the reaction was complete, the reaction product was chilled and 9.3 g of deionized water was added to the reaction vessel to dilute the reaction product. The reaction liquid was filtered using a syringe filter (pore diameter: 0.2 µm) to obtain an extract. Specifically, the extract of Comparative Example 1 was obtained by omitting the first hydrolysis step and the separation step of Experimental Example 4.

### <Comparative Example 2>

The extract of Comparative Example 2 was obtained by the same method as Comparative Example 1 except that the reaction temperature was 120 °C and the reaction time was 6 hours.

### <Comparative Example 3>

The extract of Comparative Example 3 was obtained by the same method as Comparative Example 1 except that 0.3 g of 10% (v/v) dilute sulfuric acid as the acid catalyst and 4.2 g of deionized water were added.

### <Comparative Example 4>

The extract of Comparative Example 4 was obtained by the same method as Comparative Example 2 except that 0.3 g of 10% (v/v) dilute sulfuric acid as the acid catalyst and 4.2 g of deionized water were added.

### [Measurement of Mannose Production Amount and Galactose Production Amount]

The amounts of mannose and galactose in the solution M3 separated from the reaction product M2 produced in separation step S2 and the extract M4 obtained by all of the steps including the second hydrolysis step S3 were measured using a high-performance liquid chromatograph (HPLC) (RID-10A manufactured by Shimadzu Corporation), a column (product name: Shodex SUGAR SC1011, Shodex SUGAR SC0810 manufactured by Showa Denko KK), an oven (CTO-20AC manufactured by Shimadzu Corporation), and a degasser (DGU-20A3 manufactured by Shimadzu Corporation). First, a calibration curve solution to which mannose and galactose were each added at 2 wt% was loaded into the HPLC. Thereafter, the measurement target production amounts of mannose and galactose were measured from the peak area ratios that appeared at the corresponding retention times of the HPLC. The mannose production amount was converted as a weight (mg) of mannose produced from 0.1 g of residue (mg/0.1 g).

The results of mannose extraction from the plant-based raw material coffee bean extraction residue are shown in Tables 1 to 6. The type and addition amount (wt%) of the first acid catalyst, the reaction temperature (°C), and the reaction time (h) of the first hydrolysis step are shown. Further, the amount of mannose (mg/0.1 g), the amount of galactose (mg/0.1 g), and the galactose ratio (%) contained in the solution M3 separated in the separation step are shown. The galactose ratio (%) is the ratio of the amount of galactose to the sum of the amount of galactose and the amount of mannose in the solution M3, and the amount of galactose in the solution M3 was divided by the sum of the amount of mannose and the amount of galactose to obtain a percentage. Furthermore, the type of the second acid catalyst, the reaction temperature (°C), and the reaction time of the second hydrolysis step as well as the amount of mannose (mg/0.1 g), the amount of galactose (mg/0.1 g), and the mannose ratio (%) contained in the extract after the second hydrolysis step was complete are shown as reaction results. The mannose ratio (%) is the ratio of the amount of mannose to the sum of the amount of mannose and the amount of galactose in the extract M4 after the second hydrolysis step was complete, and is the ratio of the amount of mannose in the extract M4 divided by the sum of the amount of mannose and the amount of galactose. Further, purity evaluation (A, B, C and F) for evaluating the purity of the mannose in the extract M4 after the second hydrolysis step was complete and yield evaluation (A, B, C and F) for evaluating the amount of mannose contained in the extract M4 were performed. Finally, a comprehensive evaluation (best, excellent, good, acceptable and poor) was performed based on the purity evaluation and the yield evaluation.

When 100 g of coffee bean extract residue was analyzed by high-performance liquid chromatography, mannose was 26.2 g and galactose was 9.3 g. Specifically, the component ratio of mannose to galactose in the coffee bean extract residue was approximately 74:26. Thus, in the purity evaluation, a mannose ratio of 95% or greater was evaluated as "A." A mannose ratio of 90% to less than 95% was evaluated as "B." A mannose ratio of 80% to less than 90% was evaluated as "C." A mannose ratio of less than 80% was evaluated as "F."

In the yield evaluation, a mannose production amount contained in the extract M4 of 10 mg/0.1 g or greater was evaluated as "A." 5 mg/0.1 g to less than 10 mg/0.1 g was evaluated as "B." Less than 5 mg/0.1 g is evaluated as "C."

In the comprehensive evaluation, "best" was provided if both a purity evaluation and yield evaluation were "A." "Excellent" was provided if either of the purity and yield evaluations was "A" and the other evaluation was "B." "Good" was provided if either of the purity and yield evaluations was "A" and the other evaluation was "C." "Acceptable" was provided if both the purity evaluation and yield evaluation were "C" or if either of the purity and yield evaluations was "B" and the other evaluation was "C." "Poor" was provided if either of the purity and yield evaluations was "F."

**[Table 1]**

| | | Exp Ex 1 | Exp Ex 2 | Exp Ex 3 | Exp Ex 4 | Exp Ex 5 |
|---|---|---|---|---|---|---|
| First Hydrolysis Step | First Acid Catalyst Type | Citric Acid | Citric Acid | Citric Acid | Citric Acid | Citric Acid |
| | Addition Amount (wt%) | 1.0 | 1.0 | 1.0 | 1.0 | 1.0 |
| | Temp (°C) | 120 | 120 | 120 | 120 | 120 |
| | Time (h) | 20 | 20 | 20 | 20 | 20 |
| | Mannose Production Amount (mg/0.1 g) | 4.67 | 4.67 | 4.67 | 4.67 | 4.67 |
| | Galactose Production Amount (mg/0.1 g) | 4.47 | 4.47 | 4.47 | 4.47 | 4.47 |
| | Galactose Ratio (%) | 48.91 | 48.91 | 48.91 | 48.91 | 48.91 |
| Second Hydrolysis Step | Second Acid Catalyst Type | Dilute Sulfuric Acid | Dilute Sulfuric Acid | Wood-Based Solid Acid | Wood-Based Solid Acid | Wood-Based Solid Acid |
| | Temp (°C) | 140 | 120 | 140 | 140 | 120 |
| | Time (h) | 1 | 3 | 1 | 3 | 3 |
| | Mannose Production Amount (mg/0.1 g) | 16.70 | 13.02 | 13.90 | 17.10 | 8.70 |
| | Galactose Production Amount (mg/0.1 g) | 0.60 | 1.12 | 0.50 | 0.50 | 1.10 |
| | Mannose Ratio (%) | 96.5 | 92.1 | 96.5 | 97.2 | 88.8 |
| Purity Evaluation | | A | B | A | A | C |
| Yield Evaluation | | A | A | A | A | B |
| Comprehensive Evaluation | | Best | Excellent | Best | Best | Acceptable |

**[Table 2]**

| | | Exp Ex 6 | Exp Ex 7 | Exp Ex 8 | Exp Ex 9 | Exp Ex 10 |
|---|---|---|---|---|---|---|
| First Hydrolysis Step | First Acid Catalyst Type | Citric Acid | Citric Acid | Citric Acid | Citric Acid | Citric Acid |
| | Addition Amount (wt%) | 1.0 | 10.0 | 10.0 | 10.0 | 20.0 |
| | Temp (°C) | 120 | 90 | 90 | 90 | 90 |
| | Time (h) | 20 | 24 | 48 | 72 | 24 |
| | Mannose Production Amount (mg/0.1 g) | 4.67 | 0.69 | 1.79 | 2.33 | 1.23 |
| | Galactose Production Amount (mg/0.1 g) | 4.47 | 0.44 | 1.87 | 3.02 | 1.27 |
| | Galactose Ratio (%) | 48.91 | 38.94 | 51.09 | 56.45 | 50.80 |
| Second Hydrolysis Step | Second Acid Catalyst Type | Wood-Based Solid Acid | Wood-Based Solid Acid | Wood-Based Solid Acid | Wood-Based Solid Acid | Wood-Based Solid Acid |
| | Temp (°C) | 120 | 140 | 140 | 140 | 140 |
| | Time (h) | 6 | 3 | 3 | 3 | 3 |
| | Mannose Production Amount (mg/0.1 g) | 12.50 | 19.44 | 19.83 | 18.68 | 20.66 |
| | Galactose Production Amount (mg/0.1 g) | 0.90 | 2.10 | 1.10 | 0.84 | 1.55 |
| | Mannose Ratio (%) | 93.3 | 90.3 | 94.7 | 95.7 | 93.0 |
| Purity Evaluation | | B | B | B | A | B |
| Yield Evaluation | | A | A | A | A | A |
| Comprehensive Evaluation | | Excellent | Excellent | Excellent | Best | Excellent |

**[Table 3]**

| | | Exp Ex 11 | Exp Ex 12 | Exp Ex 13 | Exp Ex 14 | Exp Ex 15 |
|---|---|---|---|---|---|---|
| First Hydrolysis Step | First Acid Catalyst Type | Citric Acid | Citric Acid | Citric Acid | Sulfuric Acid | Sulfuric Acid |
| | Addition Amount (wt%) | 30.0 | 1.0 | 1.0 | 3.7 | 1.8 |
| | Temp (°C) | 90 | 140 | 160 | 120 | 120 |
| | Time (h) | 24 | 3 | 3 | 20 | 20 |
| | Mannose Production Amount (mg/0.1 g) | 1.93 | 1.33 | 2.86 | 13.96 | 7.46 |
| | Galactose Production Amount (mg/0.1 g) | 2.08 | 3.44 | 6.40 | 6.59 | 6.88 |
| | Galactose Ratio (%) | 51.87 | 72.12 | 69.11 | 32.07 | 47.98 |
| Second Hydrolysis Step | Second Acid Catalyst Type | Wood-Based Solid Acid | Wood-Based Solid Acid | Wood-Based Solid Acid | Wood-Based Solid Acid | Wood-Based Solid Acid |
| | Temp (°C) | 140 | 140 | 140 | 140 | 140 |
| | Time (h) | 3 | 3 | 3 | 3 | 3 |
| | Mannose Production Amount (mg/0.1 g) | 18.51 | 15.50 | 10.17 | 5.76 | 11.55 |
| | Galactose Production Amount (mg/0.1 g) | 0.98 | 0.56 | 0.30 | 0.15 | 0.24 |
| | Mannose Ratio (%) | 95.0 | 96.5 | 97.1 | 97.5 | 98.0 |
| Purity Evaluation | | A | A | A | A | A |
| Yield Evaluation | | A | A | A | B | A |
| Comprehensive Evaluation | | Best | Best | Best | Excellent | Best |

**[Table 4]**

| | | Exp Ex 16 | Exp Ex 17 | Exp Ex 18 | Exp Ex 19 | Exp Ex 20 |
|---|---|---|---|---|---|---|
| First Hydrolysis Step | First Acid Catalyst Type | Sulfuric Acid | Hydrochloric Acid | Hydrochloric Acid | Acetic Acid | Acetic Acid |
| | Addition Amount (wt%) | 10.0 | 2.4 | 1.2 | 1.0 | 10.0 |
| | Temp (°C) | 120 | 120 | 120 | 140 | 140 |
| | Time (h) | 20 | 20 | 20 | 20 | 20 |
| | Mannose Production Amount (mg/0.1 g) | 15.32 | 7.42 | 0.62 | 0.82 | 2.97 |
| | Galactose Production Amount (mg/0.1 g) | 6.96 | 7.97 | 1.63 | 1.13 | 2.27 |
| | Galactose Ratio (%) | 31.24 | 51.79 | 72.44 | 57.95 | 43.32 |
| Second Hydrolysis Step | Second Acid Catalyst Type | Wood-Based Solid Acid | Wood-Based Solid Acid | Wood-Based Solid Acid | Wood-Based Solid Acid | Wood-Based Solid Acid |
| | Temp (°C) | 140 | 140 | 140 | 140 | 140 |
| | Time (h) | 3 | 3 | 3 | 3 | 3 |
| | Mannose Production Amount (mg/0.1 g) | 1.67 | 11.17 | 17.02 | 6.12 | 4.28 |
| | Galactose Production Amount (mg/0.1 g) | 0.18 | 0.27 | 0.65 | 0.15 | 0.09 |
| | Mannose Ratio (%) | 90.3 | 97.6 | 96.3 | 97.6 | 97.9 |
| Purity Evaluation | | B | A | A | A | A |
| Yield Evaluation | | C | A | A | B | C |
| Comprehensive Evaluation | | Acceptable | Best | Best | Excellent | Good |

**[Table 5]**

| | | Exp Ex 21 | Exp Ex 22 |
|---|---|---|---|
| First Hydrolysis Step | First Acid Catalyst Type | Oxalic Acid | Oxalic Acid |
| | Addition Amount (wt%) | 1.0 | 10.0 |
| | Temp (°C) | 120 | 120 |
| | Time (h) | 20 | 20 |
| | Mannose Production Amount (mg/0.1 g) | 5.03 | 16.50 |
| | Galactose Production Amount (mg/0.1 g) | 5.94 | 6.96 |
| | Galactose Ratio (%) | 54.15 | 29.67 |
| Second Hydrolysis Step | Second Acid Catalyst Type | Wood-Based Solid Acid | Wood-Based Solid Acid |
| | Temp (°C) | 140 | 140 |
| | Time (h) | 3 | 3 |
| | Mannose Production Amount (mg/0.1 g) | 9 . 53 | 0.92 |
| | Galactose Production Amount (mg/0.1 g) | 0.14 | 0.18 |
| | Mannose Ratio (%) | 98.6 | 83.6 |
| Purity Evaluation | | A | C |
| Yield Evaluation | | B | C |
| Comprehensive Evaluation | | Excellent | Acceptable |

**[Table 6]**

| | | Comp Ex 1 | Comp Ex 2 | Comp Ex 3 | Comp Ex 4 |
|---|---|---|---|---|---|
| First Hydrolysis Step | First Acid Catalyst Type | - | - | - | - |
| | Addition Amount (wt%) | | | | |
| | Temp (°C) | | | | |
| | Time (h) | | | | |
| | Mannose Production Amount (mg/0.1 g) | | | | |
| | Galactose Production Amount (mg/0.1 g) | | | | |
| | Galactose Ratio (%) | | | | |
| Second Hydrolysis Step | Second Acid Catalyst Type | Wood-Based Solid Acid | Wood-Based Solid Acid | Dilute Sulfuric Acid | Dilute Sulfuric Acid |
| | Temp (°C) | 140 | 120 | 140 | 120 |
| | Time (h) | 3 | 6 | 3 | 6 |
| | Mannose Production Amount (mg/0.1 g) | 19.50 | 15.50 | 17.33 | 18.47 |
| | Galactose Production Amount (mg/0.1 g) | 7.60 | 7.00 | 6.49 | 7.10 |
| | Mannose Ratio (%) | 72.0 | 68.9 | 72.8 | 72.2 |
| Purity Evaluation | | F | F | F | F |
| Yield Evaluation | | A | A | A | A |
| Comprehensive Evaluation | | Poor | Poor | Poor | Poor |

### [Results and Discussion]

### <Regarding First Hydrolysis Step and Separation Step>

From a comparison of all of the Experimental Examples and Comparative Examples 1 to 4, it can be understood that the purity of the ultimate mannose is significantly improved by the production method of the present invention, which includes the first hydrolysis step and the separation step. It is considered that as a result of the hydrolysis reaction in the first hydrolysis step, the galactose structure contained in the raw material coffee bean extraction residue is degraded and eluted, whereby the amount of galactose contained in the ultimate extract is reduced and the mannose ratio (purity) is increased. Thus, it has found that it is important to separate and recover the reaction product after the hydrolysis reaction in the first hydrolysis step and remove the reaction liquid (solution) after the completion of the first hydrolysis step in order to increase the purity of mannose.

### <Regarding Reaction Temperature and Reaction Time of First Hydrolysis Step>

Experimental Examples 4 and 12 will be compared. As compared with Experimental Example 4, Experimental Example 12 had a higher reaction temperature and a shorter reaction time, and in both cases, the purity and yield of the ultimately obtained mannose were evaluated better. Next, Experimental Example 12 and Experimental Example 13 will be compared. By setting the reaction temperature of Experimental Example 13 to a temperature higher than that of Experimental Example 12, the yield of the ultimately obtained mannose was reduced. It is believed that this is because the mannose structure contained in the coffee bean extraction residue was also degraded since the reaction temperature was excessively high. It can be understood that a high-purity mannose extract can be obtained even if hydrolyzation is carried out at a high temperature, but the yield of the mannose can be increased by adjusting the reaction temperature and reaction time of hydrolyzation appropriately.

Next, Experimental Examples 7 to 9 will be compared. In Experimental Examples 7 to 9, the reaction temperature was set to a temperature of 90 °C, and the reaction times were changed. Since citric acid is a weak acid and has a low hydrolysis performance, when the reaction temperature was 90 °C, which is a low temperature, Experimental Example 9, which had a long reaction time, demonstrated better results. Furthermore, When Experimental Examples 7, 10, and 11 are compared, Experimental Example 11, in which the addition amount of citric acid was high, demonstrated better results. When the reaction temperature was 90 °C and the reaction time was 24 hours, it is considered that better results were obtained by increasing the addition amount of citric acid to improve hydrolysis performance.

From these tendencies, it can be considered that the relationship between the reaction temperature and the reaction time in the first hydrolysis step is approximately inversely proportional in view of the evaluation of the purity and yield of mannose. It has been found that if citric acid is used as the first acid catalyst, suitable results can be obtained in terms of mannose purity and yield under the conditions of reaction temperature and reaction time of 120 °C for 20 hours to 140 °C for 3 hours. Furthermore, it has been found that the reaction temperature can be lowered and the reaction time can be shortened by increasing the hydrolysis performance by increasing the addition amount of citric acid as the first acid catalyst.

### <Regarding First Acid Catalyst Type>

The first acid catalyst in the first hydrolysis step will be considered. When Experimental Examples 4 and 7 to 22, in which the second acid catalyst, the reaction temperature, and the reaction time in the second hydrolysis step were the same, are compared with each other, it has been found that high-purity mannose extract can be obtained even when the first acid catalyst used is citric acid, acetic acid, or oxalic acid, which is a weak acid, or sulfuric acid or hydrochloric acid, which is a strong acid, and it has been found that in addition to citric acid, various types of acids can be used as the first acid catalyst. Furthermore, when Experimental Examples 14 to 16, Experimental Examples 17 and 18, Experimental Examples 19 and 20, and Experimental Examples 21 and 22 are compared, it has been found that the smaller the addition amount of the acid catalyst, the greater the production amount of the ultimately obtained mannose tends to increase. It is considered that, since a strong acid having a high hydrolysis performance was used as the second acid catalyst, an increase in the addition amount (the hydrolysis performance of the acid catalyst used was further increased) resulted in degradation of a part of the mannose structure which was contained in the coffee bean extraction residue in the hydrolysis of the first hydrolysis step and which was intended to be preserved in the reaction product.

### <Summary of First Hydrolysis Step>

The intent of the first hydrolysis step is to degrade and elute the galactose structure within the sugar galactomannan structure contained in the raw material. This is because the purity of the ultimately obtained mannose is considered to be increased by carrying out this step. From this, the acid catalyst used in the first hydrolysis step may be a weak acid or a strong acid. If the mannose structure is degraded and eluted in the first hydrolysis step, the amount of ultimately obtained mannose will decrease. Thus, it is preferable to appropriately adjust the addition amount, reaction temperature, and reaction time in accordance with the type of the first acid catalyst used. When increasing the addition amount of the first acid catalyst, it is preferable to reduce the reaction temperature or the reaction time. Likewise, when increasing the reaction temperature, it is preferable to reduce the reaction time or adjust the addition amount of the first acid catalyst to be smaller. When increasing the reaction time, it is preferable to reduce the reaction temperature or adjust the addition amount to be smaller. Specifically, it has been found that if the type and addition amount of the first acid catalyst and the reaction temperature and reaction time are determined so that the galactose structure within the galactomannan contained in the raw material is degraded and the mannose structure is not degraded, the purity and the yield of the ultimately obtained mannose are increased. It is considered preferable that, regarding the conditions of the first hydrolysis step, the type and the addition amount of the first acid catalyst are adjusted so that the ratio of the amount of mannose to the sum of the amount of mannose and the amount of galactose in the extract after the second hydrolysis step has completed is in the range of 80% or more and the ratio of the amount of galactose to the sum of the amount of galactose and the amount of mannose in the solution obtained in the first hydrolysis step is 38% or more, and heating is carried out at a temperature of 90 to 160 °C for 3 to 72 hours.

### (Regarding Type of Second Acid Catalyst in Second Hydrolysis Step)

The second hydrolysis step is a step in which a reaction product, from which the galactose structure is degraded and removed, is subjected to a hydrolysis treatment. Thus, the second acid catalyst used is not particularly limited. When Experimental Examples 1 and 3 and Experimental Examples 2 and 5 are compared, the ratio of mannose was substantially equal in Experimental Examples 1 and 3, which had a high reaction temperature, and the amount of mannose produced was greater in Experimental Example 1, and regarding Experimental Examples 2 and 5, both the purity and yield of mannose were superior in Experimental Example 2. This is considered to be due to the difference in the hydrolysis performance of the second acid catalyst used. It has been found that if the second acid catalyst is a strong acid having a high hydrolysis performance, degradation to mannose and extraction thereof can be sufficiently carried out in a short time at a low reaction temperature. It is considered that even if the second acid catalyst is a weak acid having a low hydrolysis performance, when the reaction temperature is increased or the reaction time is increased, degradation to mannose and extraction thereof can be sufficiently carried out. As described above, when a solid acid is used as the second acid catalyst, separation of the extraction liquid is easy, whereby an arbitrary acid catalyst can be selected depending on the extraction environment and intended use of the mannose.

### <Reaction Temperature and Reaction Time in Second Hydrolysis Step>

Next, Experimental Examples 3 and 4 and Experimental Examples 5 and 6 will be compared. The longer the reaction time, the better the purity and yield of mannose. Further, when Experimental Examples 4 and 5 are compared, the higher the reaction temperature, the better improved the purity and yield of mannose. It is considered that the mannose structure contained in the reaction product obtained by the first hydrolysis reaction was sufficiently degraded, whereby more mannose was eluted. Further, it has been found that the production amount and ratio of mannose can be improved by increasing the reaction time even at low reaction temperatures. Mannose is produced when a hydrolysis reaction is carried out on the reaction product obtained from the first hydrolysis step. Thus, like in the first hydrolysis step, in the second hydrolysis step, it is preferable to appropriately adjust the temperature range and the reaction time of the reaction temperature in accordance with the type of the acid catalyst used. When the second acid catalyst used in the second hydrolysis step is dilute sulfuric acid, it is preferable for the reaction to be carried out for a short time of about 1 hour at a high temperature of 140 °C. In the case of a wood-based solid acid, it is preferable for the reaction to be carried out for 3 hours at a high temperature of 140 °C. It is considered that even when a weak acid catalyst is used, it is possible to improve the production amount (yield) and the ratio (purity) of mannose by reacting at a high temperature for a long time.

### <Summary>

By degrading, separating, and removing galactose contained in the raw material in advance by the first hydrolysis step, high-purity mannose can be extracted in the second hydrolysis step. In the first hydrolysis step, by mainly breaking the α-1,6-glycosidic bond having a high hydrolysis rate and linking galactose and mannose together, a reaction product containing an extremely small amount of galactose and a large amount of mannose is obtained. Though the first acid catalyst used in the first hydrolysis step is not particularly limited, it is considered preferable to use a weak acid catalyst from the viewpoint of breaking the α-1,6-glycosidic bond having a high hydrolysis rate without breaking the β-1,4-glycosidic bond having a low hydrolysis rate.

The degraded galactose is eluted into the solution. Thus, by separating and removing the solution by the separation step, it is possible to separate the already degraded galactose from the reaction product. In the second hydrolysis step, high-purity mannose can be extracted by hydrolyzing the β-1,4-glycosidic bond linking mannose units together in the mannose structure contained in the reaction product. Regarding the second hydrolysis step, the second acid catalyst used is not particularly limited. In particular, the use of a solid acid catalyst is preferable because separation from the mannose extract is very easy.

As described above, when the ratio of the amount of galactose to the sum of the amount of mannose and the amount of galactose eluted in the first hydrolysis step is adjusted to be 38% or more, the yield and purity evaluation of the ultimately obtained mannose tend to be high. For example, Experimental Examples 4, 16, and 22 will be compared. Experimental Example 4, in which the ratio of galactose in the solution in the first hydrolysis step was about 49%, was excellent both in the evaluation of the purity and yield of the ultimately obtained mannose. Experimental Example 16, in which the galactose ratio was approximately 32%, and Experimental Example 22, in which it was approximately 30%, exhibited a smaller yield of mannose as compared with Experimental Example 4. Further, it is considered that although the elution amount of galactose decreased, the purity evaluation of mannose was low since the yield of mannose decreased.

As described above, high-purity mannose can be obtained by carrying out a first hydrolysis step in which a first acid catalyst is mixed with a plant-based raw material and heated, a separation step in which the reaction product obtained by the first hydrolysis step and a solution containing the components eluted by the first hydrolysis step are separated, and a second hydrolysis step in which a second acid catalyst is added to the reaction product and the mixture is heated. In particular, in the first hydrolysis step, when heating is appropriately carried out at a temperature of 90 to 160 °C for a reaction time of 3 to 72 hours, the ratio of the ultimately obtained mannose exceeds 80%, and a high-purity mannose can be obtained. It has been also found that when the ratio of the amount of galactose to the sum of the amount of galactose and the amount of mannose in the solution obtained in the first hydrolysis step is 38% or more, the yield of the ultimately obtained mannose tends to increase.

High-purity mannose is in high demand because it is used in pharmaceuticals. Mannose and galactose are similar enough in structure that they are not easy to separate, and the separation thereof is very costly and labor-intensive. Thus, the mannose extraction method of the present invention, with which high-purity mannose can be extremely easily extracted from plant-based food residues, is very useful.

### INDUSTRIAL APPLICABILITY

In the mannose extraction method of the present invention, the production of high-purity mannose from plant-based food residues is extremely easy. In particular, since no complicated processes are required and the extraction of mannose is possible by only a simple operation such as filtration separation, the design of equipment becomes easy, whereby costs are reduced. For this reason, the mannose extraction method of the present invention is highly competitive in terms of costs as compared to conventional mannose extraction methods, and is very promising as an alternative. Further, since the extracted mannose has a very high purity, it can be used for pharmaceuticals.

### DESCRIPTION OF REFERENCE SIGNS

A1 first acid catalyst
A2 second acid catalyst
C1 α-1,6-glycoside bond
C2 β-1,4-glycoside bond
GC galactose structure
GCM galactomannan structure
M1 plant-based raw material
M2 reaction product
M3 solution
M4 mannose extract
MC mannose structure
S1 first hydrolysis step
S2 separation step
S3 second hydrolysis step

## Claims

1. A mannose extraction method, wherein mannose is extracted from a plant-based raw material by carrying out:
a first hydrolysis step in which the plant-based raw material and a first acid catalyst are mixed and heated,
a separation step in which a reaction product obtained by the first hydrolysis step is separated and recovered, and
a second hydrolysis step in which the reaction product obtained by the separation step and a second acid catalyst are mixed and heated.

2. A mannose extraction method, wherein high-purity mannose is extracted from a plant-based raw material by carrying out:
a first hydrolysis step in which the plant-based raw material, which includes galactomannan, and a first acid catalyst are mixed and heated to break a bond between a galactose structure and a mannose structure in the galactomannan, and
a second hydrolysis step in which a reaction product containing the mannose structure separated by breaking from the galactose structure in the first hydrolysis step and a second acid catalyst are mixed and heated to break a bond between mannose units in the mannose structure contained in the reaction product.

3. The mannose extraction method according to claim 1 or 2, wherein the acid catalyst of the first hydrolysis step is a weak acid or dilute strong acid.

4. The mannose extraction method according to claim 3, wherein the acid catalyst of the first hydrolysis step is any of citric acid, acetic acid, oxalic acid, dilute sulfuric acid, and dilute hydrochloric acid.

5. The mannose extraction method according to any one of claims 1 to 4, wherein in the first hydrolysis step, heating is carried out for 3 to 72 hours under temperature conditions of 90 to 160 °C, and the ratio of the amount of mannose to the sum of the amount of mannose and the amount of galactose in an extract after the second hydrolysis step has completed is 80% or more.

6. The mannose extraction method according to claim 5, wherein the ratio of the amount of galactose to the sum of the amount of galactose and the amount of mannose in a solution obtained in the first hydrolysis step is 38% or more.

7. The mannose extraction method according to any one of claims 1 to 6, wherein the acid catalyst of the second hydrolysis step is any of a weak acid, a dilute strong acid, a strong acid, or a solid acid.

8. The mannose extraction method according to claim 7, wherein the acid catalyst of the second hydrolysis step is any of citric acid, acetic acid, oxalic acid, dilute sulfuric acid, dilute hydrochloric acid, sulfuric acid, hydrochloric acid, a wood solid acid catalyst obtained by introducing a sulfo group into a carbonized material derived from a wood-based raw material and sulfonating, or a resin solid acid catalyst obtained by introducing a sulfo group into a phenol resin and sulfonating.

9. The mannose extraction method according to any one of claims 1 to 8, wherein in the second hydrolysis step, heating is carried out for 1 to 24 hours under temperature conditions of 90 to 160 °C.

10. The mannose extraction method according to any one of claims 1 to 9, wherein the plant-based raw material is coffee bean extraction residue.

11. The mannose extraction method according to any one of claims 1 to 10, wherein the plant-based raw material is konjac potato.
